# EUROPEAN PATENT SPECIFICATION

(11) **EP 0 568 160 B1**
(45) Date of publication and mention of the grant of the patent: **11.06.1997**
(21) Application number: 93202062.1
(22) Date of filing: 11.10.1989
(51) Int. Cl.: A61K 7/22, A61K 7/16

(54) **Toothpaste**
Zahnpasta
Dentifrice

(30) Priority: 13.10.1988 GB 8824073; 30.11.1988 GB 8827913; 01.08.1989 GB 8917580
(43) Date of publication of application: 03.11.1993
(62) Divisional of application: 89310405.9
(73) Proprietor: BEECHAM GROUP PLC, Brentford, Middlesex TW8 9EP (GB)
(72) Inventor: Alexander, Stephen Edward SmithKline Beecham, Weybridge Surrey KT13 0DE (GB); Doel, Geoffrey Royston SmithKline Beecham, Weybridge Surrey KT13 0DE (GB); Edward, Peter John SmithKline Beecham, Weybridge Surrey KT13 0DE (GB)
(74) Representative: Connell, Anthony Christopher

(56) References cited:
- DE-A- 2 758 548
- GB-A- 2 210 265
- US-A- 4 241 049
- US-A- 4 370 314

## Description

The present invention relates to a dentifrice composition comprising an antibacterial agent, in particular an agent selected from the bis-biguanide group of antibacterials, which compositions are useful in the prophylaxis and/or treatment of periodontal disease and caries.

The bis-biguanide group of antibacterial agents have been disclosed in U.S. 2 684 924, 2 990 425, 2 830 006 and 2 863 019.

In particular, attention has focussed on two bis-biguanides viz chlorhexidine [N,N''-bis(4-chlorophenyl)-3,12-diimino-2,4,11,13-tetraazatetradecanediimidamide or 1,1'-hexamethylene-bis-[5-(p-chlorophenyl)biguanide], the compound of formula (A): 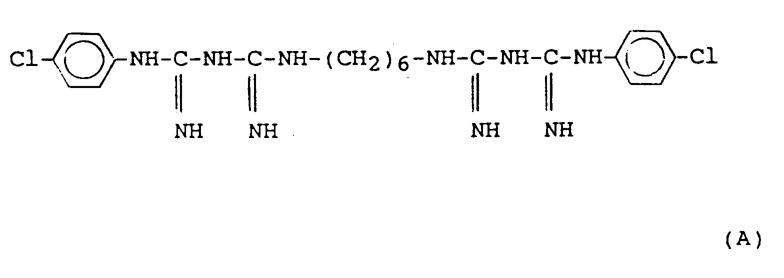 and the corresponding compound alexidene in which the chlorophenyl groups have been replaced by 2-ethylhexyl groups.

Chlorhexidine is a well established antimicrobial agent which has found use in a wide variety of applications as an antiseptic or disinfectant. In the area of oral hygiene, mouthwashes and a gel comprising chlorhexidine are commercially available. Further use of the agent has however been limited by the known propensity of chlorhexidine to form insoluble salts which remove it from solution and thereby render it unavailable. In addition, chlorhexidine has a bitter taste and tends to stain plaque brown. The latter may be minimised by using a dentifrice, rather than a mouthwash, as the abrasive incorporated therein removes plaque. The formulation of an acceptable dentifrice however provides a stern challenge, due to the multitude of components incorporated therein, each of which may be incompatible with chlorhexidine.

Various proposals have been made over the years to try and overcome the incompatibility problem, by examining various aspects of the dentifrice, for instance, the abrasive, the surfactant, the thickening agent and the process for preparation of the dentifrice.

Specific suggestions for suitable abrasives include the use of (i) abrasives coated with a cationic water soluble polymer (GB 1 506 045, to Procter + Gamble Co.), (ii) silica of a defined particle size (30% less than 5 mu) (GB 1 249 842, to Colgate-Palmolive Co.), and (iii) the use of either α-alumina monohydrate (GB 2 037 162, to Unilever NV) or α-alumina trihydrate (GB 1 302 019, to Colgate- Palmolive Co.).

Recently, EP-A-0 315 503 (to Rhone-Poulenc Chimie) (published after the earliest priority date of the present application) has disclosed the suitability of particular types of silicas, characterised by inter alia, a low anion (<1%) content. Whilst the compatibility of these silicas with chlorhexidine and with other dentifrice ingredients was established by a spectrophotometric assay, no consideration was given to the compatibility of chlorhexidine with the other ingredients.

Specific suggestions for suitable surfactants include the use of either an amphoteric surfactant (in combination with a nonionic thickening agent such as hydroxyethyl cellulose) (JP 51 051 530, to Lion Fat and Oil KK); or a polyoxyethylene fatty ester to augment an anionic surfactant (in combination with an anionic thickening agent, such as sodium carboxymethyl cellulose) (JP 50 076 243, to Sunstar Dentifrice KK). The natural gum carrageenan was suggested to be a suitable thickening agent (JP 79 011 243, Lion Dentifrice Co.).

It has also been suggested that either ethanol or a water soluble alkaline earth metal salt (when the dentifrice also includes a phosphate salt) may be added, to stabilise chlorhexidine in a dentifrice comprising otherwise conventional components (US 3 989 813 and US 4 241 049, both to Colgate-Palmolive Co.). Similarly, the use of a phenol or a higher alcohol has been proposed, to stabilise a dentifrice comprising chlorhexidine and a nonionic surfactant (JP 59 101 417, JP 59 101 418, both to Lion Corporation).

US 4 273 759 (to Colgate-Palmolive Co.) discloses a chlorhexidine dentifrice comprising hydrated alumina, a nonionic surfactant and hydroxypropyl methylcellulose.

FR 2 341 302 (to Pierre Fabre SA) discloses the use of a microbiological assay for assessing the compatibility of chlorhexidine with various types of conventional dentifrice ingredient. Anionic surfactants, the abrasive bentonite and carragheen and alginate gums were found to be unsuitable.

The appropriate sequence of addition of the various components has been identified as being of importance in the maintainance of chlorhexidine in a soluble form in the formulation (US 3 843 779 and US 3 842 168, both to Colgate-Palmolive Co., and JP 60 130 511, to Lion Coporation).

Several methods of overcoming the unpleasant bitter flavour associated with chlorhexidine have been disclosed, including the use, in an alumina-based dentifrice, of a predominantly peppermint flavour, moderated by the addition of an aniseed flavour (GB 2 035 084, to Unilever).

In spite of all these proposals, as far as we are aware, no dentifrice comprising an effective amount of chlorhexidine or any other bis-biguanide antibacterial agent is currently available commercially.

It has now been discovered that this problem of incompatability can be overcome or at least mitigated by the use of a particular formulation of dentifrice.

Accordingly, the present invention provides a dentifrice comprising:
(a) a bacteriostatically effective amount of a bis-biguanide compound of formula (I): 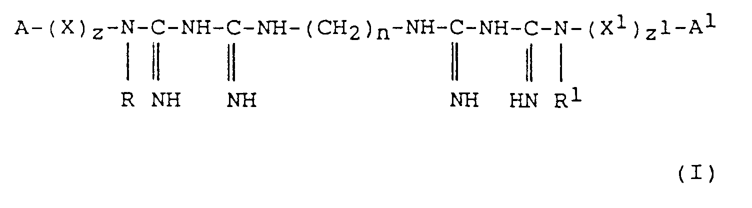 wherein:
   A and A¹ each represent (i) a phenyl group optionally substituted by (C₁₋₄)alkyl, (C₁₋₄)alkoxy, nitro, or halogen, (ii) a (C₁₋₁₂)alkyl group, or (iii) a (C₄₋₁₂) alicyclic group;
   X and X¹ each represent (C₁₋₃)alkylene;
   R and R¹ each represent hydrogen, (C₁₋₁₂)alkyl, or aryl(C₁₋₆)alkyl;
   Z and Z¹ are each O or 1;
   n is an integer from 2 to 12;
   and the polymethylene chain (CH₂)ₙ may optionally be interrupted by oxygen or sulphur or an aromatic (for instance phenyl or naphthyl) nucleus;
   or an orally acceptable acid addition salt thereof, in aqueous solution;
(b) a nonionic thickening agent;
(c) a nonionic surfactant; and
(d) an abrasive selected from calcium carbonate, calcium phosphate, magnesium carbonate, insoluble sodium metaphosphate or suitable mixtures thereof, in combination with an agent to suppress anion formation; or

Advantageously, the bis-biguanide of formula (I) is present in the range 0.005 to 10%, preferably 0.005 to 5%, more preferably 0.005 to 2.5% by weight of the dentifrice, calculated as the free base.

Examples of bis-biguanides of formula (I) are chlorhexidine and alexidine, of which chlorhexidine is particularly preferred.

Suitable acid addition salts of the bis-biguanides of formula (I) include the diacetate, the dihydrochloride and the digluconate.

Suitable acid addition salts of chlorhexidine are those which have a water solubility at 20^{o}C of at least 0.005% w/v and include the digluconate, diformate, diacetate, dipropionate, dihydrochloride, dihydroiodide, dilactate, dinitrate, sulphate, and tartrate salts. Preferably the salt is the dihydrochloride, diacetate or digluconate salt.

Suitable acid addition salts of alexidine include the dihydrochloride salt.

Suitable nonionic thickening agents include (C₁₋₆)alkylcellulose ethers, for instance methylcellulose, and (C₂₋₆)alkylene oxide modified (C₁₋₆)alkylcellulose ethers, for instance hydroxypropyl methylcellulose, and mixtures thereof.

Preferably, the nonionic thickening agent is a low viscosity grade of hydroxypropyl methylcellulose which may advantageously be used in combination with a high viscosity grade of hydroxypropyl methylcellulose, the mixture being balanced to give the formulation the required viscosity.

Suitable grades of hydroxypropyl methylcellulose are marketed under the trade name 'Methocel' by Dow Chemical Corporation. The grades 'Methocel K15M' (high viscosity) and 'Methocel K100LV' are found to be particularly useful, an effective ratio thereof being in the range of from 1:50 to 1:1, preferably 1:20 to 1:2.

Advantageously, the nonionic thickening agent is present in the range 0.01 to 30%, preferably 0.1 to 15%, more preferably 1 to 5%, by weight of the dentifrice.

Suitable nonionic surfactants include, for instance polyethoxylated sorbitol monoesters (for instance, the products marketed under the trade name 'Tween' by ICI), polycondensates of ethylene oxide and propylene oxide (poloxamers) (for instance the products marketed under the trade name 'Pluronic' by BASF-Wyandotte), condensates of propylene glycol and polyethoxylated hydrogenated castor oil (for instance, cremophors).

Advantageously, the surfactant is present in the range 0.005 to 20%, preferably 0.1 to 10%, more preferably 0.1 to 5% by weight of the dentifrice.

The sparingly soluble salts used as an abrasive include calcium carbonate, calcium phosphates, magnesium carbonate, insoluble sodium metaphosphate, and suitable mixtures thereof. The agent to suppress anion formation typically comprises a water soluble salt containing a cation which may be same as the cation of the abrasive and which forms an essentially insoluble or sparingly soluble salt with the anion of the abrasive.

Preferably the sparingly soluble salt used as an abrasive is calcium carbonate, advantageously used in combination with dicalcium phosphate, which also usefully buffers the pH of the formulation. Suitable types of calcium carbonate include both natural and synthetic chalks.

The agent to suppress anion formation may be an alkaline earth metal salt, for instance calcium chloride. The agent is preferably present in from 0.0001 to 1%, more preferably 0.005 to 0.1% by weight of the dentifrice.

Dentifrices according to the invention may also contain a humectant, such as glycerine, sorbitol, propylene glycol or polyethylene glycol, or mixtures thereof; which humectant may be present in the range 5 to 30%, preferably 10 to 30% by weight of the dentifrice. Preferably the humectant is glycerine.

Dentifrices according to the invention may also contain other agents conventionally used in dentifrice formulations, for instance flavouring agents; colouring agents; whitening agents; preservatives and sweetening agents. It will be a appreciated that such agents (at the level employed) should be compatible with the bis-biguanide of formula (I); that is, the agent will not substantially reduce the availability of the bis-biguanide of formula (I). This may be confirmed by, for instance, determining the biological activity of the formulation, (by following the method provided in the 'Biological Data' section) in the presence and absence of the agent. In general, such agents will be a minor amount or proportion of the formulation, usually present in from 0.001 to 5% by weight of the composition.

Flavour is an important aspect of the consumer acceptability of a dentifrice. This is particularly so in the case of a dentifrice comprising a bis-biguanide of formula I, especially chlorhexidine, because of the bitter after-taste of the bis-biguanide. Surprisingly it has now been found that this can be effectively masked by an aniseed flavour.

Accordingly, in a further aspect, the present invention provides a dentifrice as hereinbefore defined which further comprises a flavouring agent having an aniseed flavour.

Flavouring agents having an aniseed flavour include anethole, which may be present in such quantity as to mask the bitter after-taste of the bis-biquanide of formula (I).

Preferably the flavour may be modified by the additional incorporation of one or more flavouring agents having a mint flavour, to balance the aniseed flavour, to give a flavour which has more general consumer acceptability but in which the aniseed flavour is still dominant.

Suitable flavouring agents having a mint flavour include peppermint, spearmint, menthol and carvone.

Preferably more than one mint flavouring agent is used, of which menthol may be the major component, accounting for between 20 and 60%, preferably between 25 and 55% by weight of the flavouring agents having a mint flavour.

Advantageously the flavour of the dentifrice may be further modified by the incorporation of flavouring agents having spicey flavours such as coriander, eugenol and eucalyptol, the aniseed flavour still being dominant.

Accordingly, in a still further aspect, the invention provides a dentifrice as hereinbefore defined comprising aniseed and mint flavours and, optionally, spicey flavours, and having a predominantly aniseed flavour.

Preferably, in such a dentifrice comprising aniseed and mint flavours, flavouring agents having an aniseed flavour comprise from 10 to 30%, more preferably 15 to 25% of the combined weights of the flavouring agents, whilst flavouring agents having a mint flavour comprise from 40 to 80, preferably 40 to 70% of the combined weight of the flavouring agents. Preferably the combined flavouring agents comprise upto 5%, more preferably upto 2% by weight of the dentifrice.

Suitable sweetening agents include saccharin and acceptable water soluble salts thereof, such as the sodium salt, and may be present in from 0.01 to 0.5%, preferably 0.05 to 0.5% by weight of the dentifrice. An auxiliary sweetener such as a thaumatin may also be included, at a level of from 0.001 to 0.1, preferably 0.005 to 0.05% by weight of the dentifrice. A suitable blend of thaumatins is marketed under the trade name 'Talin' by Tate and Lyle plc.

Accordingly, in a preferred aspect, the invention provides a dentifrice as hereinbefore defined, comprising aniseed and mint flavours, in combination with saccharin, or the sodium salt thereof, and a thaumatin.

Water, preferably deionised or distilled water, will also be present, in the range from 10 to 80%, preferably 20 to 70% by weight of the dentifrice.

The dentifrices according to the invention may have a pH within the range pH 4 to 10, preferably pH 5 to 8.

In an especially preferred aspect, the invention provides a dentifrice comprising:
(i) chlorhexidine or an acid addition salt thereof;
(ii) a (C₂₋₆)alkylene oxide modified (C₁₋₆)alkyl cellulose thickening agent;
(iii) a poloxamer surfactant; and
(iv) an abrasive which is calcium carbonate, optionally in combination with dicalcium orthophosphate and comprising an alkaline earth salt to act as an anion suppressant agent.

The dentifrices according to the invention are prepared in a conventional manner by mixing the ingredients thereof in the required proportions and in any order which is convenient and, thereafter and if necessary, adjusting the pH. For instance, the nonionic thickening agent and the humectant and part of the water are vigourously agitated together, with heat, if necessary, to give a hydrated gel. Abrasive is then dispersed in this hydrated gel, using a heavy-duty mixing machine, with active agents, such as chlorhexidine, or a salt thereof, a fluoride salt (if present), then added, followed by nonionic surfactant and flavouring agents in the final stage; with final mixing carried out under vacuum.

The following examples illustrate the invention.

### Example 1

| Chalk Based Toothpaste | |
|---|---|
| Chlorhexidine digluconate | 1.00% |
| Glycerin | 18.00 |
| Hydroxypropyl methylcellulose | 3.60 |
| Chalk | 32.00 |
| Dicalcium phosphate dihydrate | 3.00 |
| Calcium chloride solution (1%) | 1.00 |
| Flavour | 1.00 |
| Poloxamer 338 | 2.00 |
| Deionized water | q.s. |

### Biological Data

The data in the following table represents the potency of chlorhexidine in the dentifrice formulation, compared with a control containing chlorhexidine digluconate alone in aqueous solution and in the absence of the other agents listed. The assay is a standard agar diffusion method, using M. luteus as the assay organism.

| | Potency % |
|---|---|
| Example 1 | 79 |
| Control | 100 |

The data show that when chlorhexidine is incorporated into a dentifrice formulation according to the present invention, chlorhexidine substantially retains its potency, thereby allowing it to exert its intrinsic bacteriostatic activity within the oral cavity.

## Claims

1. A dentifrice comprising:
(a) a bacteriostatically effective amount of a bis-biguanide compound of formula (I): 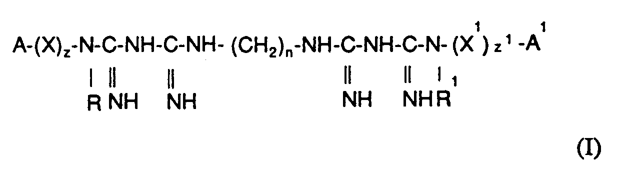 wherein:
A and A¹ each represent (i) a phenyl group optionally substituted by (C₁-₄)alkyl, (C₁-₄)alkoxy, nitro, or halogen, (ii) a (C₁-₁₂)alkyl group, or (iii) a (C₄-₁₂)-alicyclic group;
X and X¹ each represent (C₁-₃)alkylene;
R and R¹ each represent hydrogen, (C₁-₁₂)alkyl, or aryl(C₁-₆)alkyl;
Z and Z¹ are each 0 or 1;
n is an integer from 2 to 12;
and the polymethylene chain (CH₂)ₙ may optionally be interrupted by oxygen or sulphur or an aromatic nucleus;
or an orally acceptable acid addition salt thereof, in aqueous solution;
(b) a nonionic thickening agent;
(c) a nonionic surfactant; and
(d) an abrasive selected from calcium carbonate, calcium phosphate, magnesium carbonate, insoluble sodium metaphosphate or suitable mixtures thereof, in combination with an agent to suppress anion formation

2. A dentifrice as claimed in claim 1 in which the bis-biguanide of formula (I) is chlorhexidine or alexidine, or an orally acceptable acid addition salt thereof.

3. A dentifrice as claimed in claim 1 or claim 2 in which the nonionic thickening agent is a (C₁-₆)alkyl cellulose ether, or a (C₂-₆)alkylene oxide modified (C₁-₆)alkylcellulose ether, or a mixture thereof.

4. A dentifrice as claimed in any one of claims 1 to 3 in which the abrasive comprises calcium carbonate, optionally used in combination with dicalcium orthophosphate.

5. A dentifrice as claimed in any one of claims 1 to 4 in which the agent to suppress anion formation comprises a water soluble salt containing a cation which is optionally the same as the cation of the abrasive and which forms an essentially insoluble or sparingly soluble salt with the anion of the abrasive.

6. A dentifrice as claimed in any one of claims 1 to 5 comprising flavouring agents having an aniseed and, optionally, a mint and a spicey flavour, the dentifrice having a predominantly aniseed flavour.

7. A dentifrice comprising:
(i) chlorhexidine or an acid addition salt thereof;
(ii) a (C₂₋₆)alkylene oxide modified (C₁₋₆)alkyl cellulose thickening agent;
(iii) a poloxamer surfactant; and
(iv) an abrasive which is calcium carbonate, optionally in combination with dicalcium orthophosphate and comprising an alkaline earth salt to act as an anion suppressant agent.

8. A dentifrice as claimed in any one of claims 1 to 7 for use in oral hygiene.

9. A process for preparing a dentifrice as defined in any one of claims 1 to 7 which process comprises admixing the ingredients in appropriate quantities, in any order that is convenient, and thereafter, and if necessary, adjusting the final pH of the dentifrice.

## Patentansprüche

1. Zahnputzmittel, enthaltend:
(a) eine bakteriostatisch wirksame Menge einer Bisbiguanidverbindung der Formel (I): 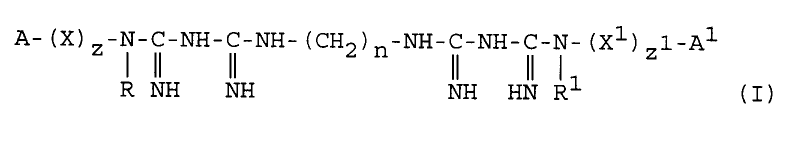 worin:
A und A¹ jeweils darstellen:
(i) eine Phenylgruppe, gegebenenfalls substituiert mit einer (C₁₋₄)Alkyl-, (C₁₋₄)Alkoxy- oder Nitrogruppe oder einem Halogenatom,
(ii) einen (C₁₋₁₂)Alkylrest oder
(iii) einen (C₄₋₁₂)alicyclischen Rest;
X und X¹ jeweils (C₁₋₃)-Alkylenreste darstellen;
R und R¹ jeweils ein Wasserstoffatom, einen (C₁₋₁₂)-Alkyl- oder Aryl(C₁₋₆)alkyl-Rest darstellen;
Z und Z¹ jeweils 0 oder 1 sind;
n eine ganze Zahl von 2 bis 12 ist;
und die Polymethylenkette (CH₂)ₙ gegebenenfalls von Sauerstoff oder Schwefel oder einem aromatischen Kern unterbrochen sein kann;
oder dessen mundverträgliches Säureadditionssalz in wäßriger Lösung;
(b) ein nichtionisches Dickungsmittel;
(c) ein nichtionisches Tensid, und
(d) ein Abriebmittel gewählt aus Calciumcarbonat, Calciumphosphat, Magnesiumcarbonat, unlöslichem Natriummetaphosphat oder geeigneten Mischungen davon, in Kombination mit einem die Bildung von Anionen unterdrückendem Mittel.

2. Zahnputzmittel gemäß Anspruch 1, in welchem das Bisbiguanid der Formel I Chlorhexidin oder Alexidin ist, oder dessen mundverträgliches Säureadditionssalz.

3. Zahnputzmittel gemäß den Ansprüchen 1 oder 2, in welchem das nichtionische Dickungsmittel ein (C₁₋₆)Alkylcelluloseether oder ein (C₂₋₆)alkylenoxid-modifizierter (C₁₋₆)-Alkylcelluloseether, oder eine Mischung daraus ist.

4. Zahnputzmittel gemäß einem der Ansprüche 1 bis 3, in welchem das Abriebmittel Calciumcarbonat umfaßt, das gegebenenfalls in Kombination mit Dicalciumorthophosphat verwendet wird.

5. Zahnputzmittel gemäß einem der Ansprüche 1 bis 4, in welchem das die Bildung von Anionen unterdrückende Mittel ein wasserlösliches Salz umfaßt, das ein Kation enthält, das gegebenenfalls dasselbe Kation ist, wie das des Abriebmittels, und das ein im wesentlichen unlösliches oder schwerlösliches Salz mit dem Anion des Abriebmittels bildet.

6. Zahnputzmittel gemäß einem der Ansprüche 1 bis 5, umfassend Geschmacksstoffe, die einen anisartig und wahlweise einen minzigen oder würzigen Geschmack besitzen, wobei das Zahnputzmittel einen überwiegenden anisartigen Geschmack hat.

7. Zahnputzmittel, umfassend
(i) Chlorhexidin oder ein Säureadditionssalz davon;
(ii) ein (C₂₋₆)alkylenoxid-modifiziertes (C₁₋₆)Alkylcellulosedickungsmittel;
(iii) ein poloxameres Tensid; und
(iv) ein Abriebmittel, welches Calciumcarbonat, gegebenenfalls in Kombination mit Dicalciumorthophosphat, ist und ein Erdalkalisalz enthält, das als Anionen unterdrückendes Mittel wirkt.

8. Zahnputzmittel gemäß einem der Ansprüche 1 bis 7 zur Verwendung in der Mundhygiene.

9. Verfahren zur Herstellung eines Zahnputzmittels gemäß einem der Ansprüche 1 bis 7, umfassend das Vermischen der Bestandteile in zweckmäßigen Mengen in jeder geeigneten Reihenfolge und danach, und falls nötig, das Einstellen des End-pH-Wertes des Zahnputzmittels.

## Revendications

1. Dentifrice contenant:
(a) une quantité efficace du point de vue bactériostatique, d'un composé bis-biguanide de formule (I) : 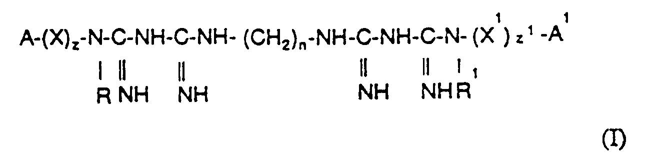 dans laquelle :
- A et A¹ représentent (i) un groupe phényle éventuellement substitué par un groupe alkyle (C₁₋₄), alcoxy (C₁₋₄), nitro, ou halogène, (ii) un groupe alkyle (C₁₋₁₂), ou (iii) un groupe alicyclique (C₄₋₁₂);
- X et X¹ représentent chacun un groupe alkylène (C₁₋₃);
- R et R¹ représentent chacun un atome d'hydrogène, un groupe alkyle (C₁₋₂) ou arylalkyle (C₁₋₆);
- Z et Z¹ sont chacun égaux à 0 ou à 1;
- n est un entier variant de 2 à 12;
- et la chaîne polyméthylénique (CH₂)ₙ peut éventuellement être interrompue par un atome d'oxygène, un atome de soufre, ou un noyau aromatique;
ou un sel d'addition d'acide de celui-ci, acceptable par voie orale, en solution aqueuse;
(b) un agent épaississant non ionique;
(c) un surfactant non ionique ; et
(d) un abrasif choisi parmi le carbonate de calcium, le phosphate de calcium, le carbonate de magnésium, le métaphosphate de sodium insoluble, ou des mélanges appropriés de ceux-ci, en combinaison avec un agent supprimant la formation d'anion.

2. Dentifrice selon la revendication 1, dans lequel le bisbiguanide de formule (I) est la chlorhexidine ou l'alexidine, ou un sel d'addition d'acide de celles-ci, acceptable par voie orale.

3. Dentifrice selon la revendication 1, ou la revendication 2, dans lequel l'agent épaississant non ionique est un alkyl (C₁₋₆) cellulose éther, ou un alkyl (C₁₋₆) cellulose éther modifié alkylène (C₂₋₆) oxyde, ou un mélange de ceux-ci.

4. Dentifrice selon l'une quelconque des revendications 1 à 3, dans lequel l'abrasif comprend le carbonate de calcium, éventuellement utilisé en combinaison avec l'orthophosphate dicalcique.

5. Dentifrice selon l'une quelconque des revendications 1 à 4, dans lequel l'agent supprimant la formation d'anion comprend un sel hydrosoluble contenant un cation qui est éventuellement le même que celui de l'abrasif, et qui forme avec l'anion de l'abrasif, un sel pratiquement insoluble, ou peu soluble.

6. Dentifrice selon l'une quelconque des revendications 1 à 5, contenant des agents aromatisants ayant un goût anisé, et éventuellement un goût de menthe et épicé, le dentifrice étant de façon prédominante, de goût anisé.

7. Dentifrice comprenant :
(i) de la chlorhexidine ou un sel d'addition d'acide de celle-ci;
(ii) un agent épaississant alkyl (C₁₋₆) cellulose éther modifié alkylène (C₂₋₆)oxyde;
(iii) un surfactant poloxamer; et
(iv) un abrasif qui est le carbonate de calcium, éventuellement en combinaison avec de l'orthophosphate dicalcique, et contenant un sel alcalinoterreux agissant comme agent suppresseur d'anion.

8. Dentifrice selon l'une quelconque des revendications 1 à 7, destiné à l'utilisation en hygiène buccale.

9. Procédé de préparation d'un dentifrice selon l'une quelconque des revendications 1 à 7, lequel procédé comprend le mélange des ingrédients en quantités appropriées, dans un ordre convenable quelconque, et ensuite, et si nécessaire, l'ajustage du pH final du dentifrice.
